# EUROPEAN PATENT APPLICATION

(11) **EP 4 349 444 A1**
(43) Date of publication of application: **10.04.2024**
(21) Application number: 22816118.8
(22) Date of filing: 31.05.2022
(51) Int. Cl.: B01D 9/02, C07C 51/43, C07C 57/055

(54) **PURIFICATION APPARATUS**

(30) Priority: 02.06.2021 JP 2021093031
(71) Applicant: Nippon Shokubai Co., Ltd., Osaka-shi, Osaka 541-0043 (JP)
(72) Inventor: FUKUMOTO, Toshiya, Himeji-shi, Hyogo 671-1282 (JP); MUKAE, Masashi, Himeji-shi, Hyogo 671-1282 (JP); MATSUDA, Takayuki, Himeji-shi, Hyogo 671-1282 (JP)
(74) Representative: Schön, Christoph
(86) International application number: PCT/JP2022/022160
(87) International publication number: WO 2022/255372

(57) **Abstract**

Provided is a method for stably obtaining a product. The present invention relates to a purification apparatus for purifying a compound, the purification apparatus including: a tank for use in the purification apparatus; and a hydraulic wash column, the tank being at least one of a crystallization tank that forms a slurry containing crystals of a compound or a ripening tank that is capable of keeping crystals of a compound suspended in the ripening tank, the tank including an agitator having an agitator shaft and a bearing, the purification apparatus further including:
a line for discharging the slurry containing crystals of a compound from the tank and feeding the slurry to the hydraulic wash column; and a line for flowing between the agitator shaft and the bearing in the tank at least one of a mother liquor derived from the slurry fed to the hydraulic wash column, the slurry containing crystals of a compound, or a melt obtained by melting the crystals.

## Description

### TECHNICAL FIELD

The present invention relates to a purification apparatus. Specifically, the present invention relates to a purification apparatus, a method for producing a compound, and a method for purifying a compound.

### BACKGROUND ART

Purification apparatuses have been widely used industrially to purify compounds that are used as raw materials for resins, for example. In many fields of the chemical industry, high-quality compounds with reduced impurities have been required, and for such compounds, various studies have been conducted on better purification apparatuses.

Industrially, many of crude compounds, which are compounds before purification, are purified through continuous purification processes. Disclosed is a method for producing acrylic acid including: collecting and crystallization purifying a gas containing acrylic acid obtained by catalytic vapor phase oxidation of a raw material gas; and returning acrylic acid obtained by decomposing a substance obtained by Michael addition of acrylic acid in a residual mother liquor to the collecting step, for example (see, for example, Patent Literature 1).

In order to obtain a high-purity compound in a higher yield, the purification uses a tank that forms a slurry containing crystals of a compound (crystallization tank) and/or a tank that grows the crystals of a compound (ripening tank). Patent Literatures 2 to 4 disclose conventional purification methods using a crystallization tank and/or a ripening tank.

### CITATION LIST

### - Patent Literature

Patent Literature 1: JP 2007-182437 A
Patent Literature 2: JP 2005-28214 A
Patent Literature 3: JP 2012-140471 A
Patent Literature 4: JP 2002-204937 A

### SUMMARY OF INVENTION

### - Technical Problem

As described above, better purification apparatuses for producing compounds have been desired, and methods for stably obtaining products (compounds) have been desired. The present invention has been made in view of the above-mentioned current state of the art, and aims to provide a method for stably obtaining a product.

### - Solution to Problem

The present inventors have studied a method for stably obtaining a product, and focused on a purification apparatus including a tank for use in the purification apparatus and a hydraulic wash column. Specifically, the present inventors focused on a purification apparatus including: a tank that is at least one of a crystallization tank that forms a slurry containing crystals of a compound or a ripening tank that is capable of keeping crystals of a compound suspended in the ripening tank and that includes an agitator having an agitator shaft and a bearing; a line for discharging the slurry containing crystals of a compound from the tank and feeding the slurry to a hydraulic wash column; and a line for flowing between the agitator shaft and the bearing in the tank at least one of a mother liquor derived from the slurry fed to the hydraulic wash column, the slurry containing crystals of a compound, or a melt obtained by melting the crystals. The present inventors found that during use of such a tank, the crystal-containing slurry can be prevented from entering between the rotating agitator shaft and the stationary bearing; wear of the agitator shaft and the bearing due to the crystals can be prevented; and when the compound is a polymerizable substance, polymerization and freezing due to sliding between the agitator shaft and the bearing can be sufficiently prevented. Thereby, a product can be stably obtained. Thus, the present invention has been completed.

That is, the present invention relates to a purification apparatus for purifying a compound, the purification apparatus including:
a tank for use in the purification apparatus; and
a hydraulic wash column,
the tank being at least one of a crystallization tank that forms a slurry containing crystals of a compound or a ripening tank that is capable of keeping crystals of a compound suspended in the ripening tank,
the tank including an agitator having an agitator shaft and a bearing,
the purification apparatus further including:
   a line for discharging the slurry containing crystals of a compound from the tank and feeding the slurry to the hydraulic wash column; and
   a line for flowing between the agitator shaft and the bearing in the tank at least one of a mother liquor derived from the slurry fed to the hydraulic wash column, the slurry containing crystals of a compound, or a melt obtained by melting the crystals.

### - Advantageous Effects of Invention

The purification apparatus of the present invention can be stably operated for a long time, whereby the productivity can be increased and the maintenance costs can be reduced.

### BRIEF DESCRIPTION OF DRAWINGS

FIG. 1 is a schematic cross-sectional side view of an exemplary tank in a purification apparatus of the present invention.
FIG. 2a is a schematic diagram of exemplary lines to which the present invention is applicable, in the purification apparatus of the present invention.
FIG. 2b is a schematic diagram of other exemplary lines to which the present invention is applicable, in the purification apparatus of the present invention.
FIG. 3 is a schematic side view of part of an agitator in the tank shown in FIG. 1.
FIG. 4 is a schematic cross-sectional side view of a bearing of the agitator in the tank shown in FIG. 1.
FIG. 5 is a schematic diagram of other exemplary lines to which the present invention is applicable, in the purification apparatus of the present invention.

### DESCRIPTION OF EMBODIMENTS

The present invention will be described in detail below.

It should be noted that combinations of two or more of the preferred features of the present invention described below are also preferred embodiments of the present invention.

The following first describes a purification apparatus of the present invention, followed by descriptions of a method for producing a compound of the present invention and a method for purifying a compound of the present invention in this order.

### (Purification apparatus of the present invention)

The purification apparatus of the present invention includes a tank for use in the purification apparatus and a hydraulic wash column. The purification apparatus of the present invention further includes the lines described above. The following first describes the tank, followed by the hydraulic wash column, the lines, and other units.

### <Tank>

The tank is a crystallization tank that forms a slurry containing crystals of a compound and/or a ripening tank that is capable of keeping crystals of a compound suspended in the ripening tank. The ripening tank keeps the crystals of a compound therein for a certain period of time to grow the crystals of a compound.

The tank includes an agitator having an agitator shaft and a bearing in the tank.

The bearing accommodates at least part of the agitator shaft of the agitator, with a space between the bearing and the agitator shaft. The bearing may or may not be in contact with at least part of the agitator shaft of the agitator.

When the bearing is in contact with at least part of the agitator shaft of the agitator, it may be in contact with at least part of the agitator shaft of the agitator directly or via a ball, roller (rod), or shaft sleeve.

For example, preferably, the bearing accommodates and supports the tip of the agitator shaft of the agitator.

Herein, the inner side surrounded by a bottom part and/or a side part of the bearing and configured to accommodate at least part of the agitator shaft is also referred to as an inner side of the bearing.

The direction of the agitator shaft of the agitator is not limited. The agitator shaft is preferably provided in the direction from the top-roof side to the bottom side of the tank. When the agitator shaft is provided in the direction from the top-roof side to the bottom side of the tank, the agitator shaft does not have to be in contact with the top roof and the bottom of the tank, and the direction (axial direction) needs to be the direction from the top-roof side to the bottom side of the tank.

In particular, the agitator shaft of the agitator is preferably provided such that the direction thereof forms an angle within the range of 0° to 30° with the vertical direction, still more preferably within the range of 0° to 15°, further preferably within the range of 0° to 10°, particularly preferably within the range of 0° to 5°. Most preferably, the agitator shaft is provided in the vertical direction.

Preferably, the bearing includes, for example, a bottom part covering the bottom face of the tip of the agitator shaft and/or a side part covering the side face of the tip of the agitator shaft. For example, the bearing may consist of a side part covering the side face of the tip of the agitator shaft. In this case, the lower side (bottom side) of the agitator shaft is not covered with the bearing and the bearing does not have a structure in which liquid or slurry is accumulated. The effects of the present invention can still be exhibited by flowing the mother liquor and/or melt in the space between the bearing and the side face of the tip of the agitator shaft to prevent the crystal-containing slurry from entering the space. The side part of the bearing may be a ring having a ring shape, for example.

The upper side of the bearing is partly or entirely uncovered. The liquid in the bearing can be overflowed from the upper side.

The bottom part may be inclined with respect to the horizontal direction, and is preferably horizontal.

The size of the bearing is not limited, and can be appropriately selected according to the size of the agitator. For example, the bearing preferably has an inner diameter of 10 to 500 mm.

The inner diameter of the bearing is preferably 1/1000 or more, more preferably 1/800 or more, of the inner diameter of the tank when the tank is viewed from the top.

The inner diameter of the bearing is preferably 1/5 or less of the inner diameter of the tank.

Herein, the inner diameter of the bearing is the inner diameter of a portion of the bearing covering the side face of the agitator shaft when the bearing is viewed from the direction of the agitator shaft. When the inner side of the portion has a shape different from a circular shape, the inner diameter of the bearing refers to the maximum distance (distance on the horizontal plane) between two points on the contour corresponding to the inner side of the portion.

The inner diameter of the tank refers to the inner diameter when the tank is viewed from the top. When the tank has a shape different from a cylindrical shape, the inner diameter of the tank refers to the maximum distance (distance on the horizontal plane) between two points on the contour corresponding to the inner wall surface of the tank when the tank is viewed from the top.

The inner height (depth) of the bearing is not limited and can be appropriately selected according to the size of the agitator. For example, it is preferably 30 to 500 mm.

The inner height of the bearing is preferably 1/1000 or more, more preferably 1/800 or more, still more preferably 1/500 or more, of the inner height of the tank.

The inner height of the bearing is preferably 1/5 or less, more preferably 1/10 or less, of the inner height of the tank.

Herein, the inner height of the bearing refers to the average height of the entire inner side of the bearing. When the bearing does not have an upper part and/or a bottom part, the inner height of the bearing corresponds to the height of an inner side part of the bearing.

The inner height of the tank refers to the difference between the average height of the entire top surface in the tank and the average height of the entire bottom in the tank.

The distance (shortest distance) between the agitator shaft and the bearing is preferably in the range of 0.01 to 50 mm, more preferably in the range of 0.05 to 10 mm.

The bearing may be made of any material. Examples of the material include metals such as stainless steel, resins, carbon-based materials such as carbon fiber, glass, and mixtures of these materials. Preferred examples thereof include carbon-based materials, metals containing carbon-based materials, and Teflon (registered trademark) containing glass.

When an agitator is used during use of the tank, a suspension part can be formed in at least a part of the tank. In the suspension part in the tank, crystals can be kept for a certain period of time, and after the crystals have grown sufficiently, they can be discharged as a slurry or the like from a portion in the vicinity of the bottom of the tank, for example. For example, in the ripening tank, when the crystals are kept for a certain period of time, fine crystals are melted by Ostwald ripening and large crystals grow further, so that the crystal size distribution becomes narrow. As a result, high-quality crystals can be obtained, and by subjecting such crystals to the subsequent purification in the hydraulic wash column, the purification efficiency in the hydraulic wash column can be further improved. Even in the crystallization tank, the same effect as in the ripening tank can be expected by keeping the crystals for a certain period of time.

In the tank, a mother liquor derived from the slurry fed to the hydraulic wash column, the slurry containing crystals of a compound, and/or a melt obtained by melting the crystals are flowed between the agitator shaft and the bearing in the tank. Thereby, the crystal-containing slurry can be prevented from entering between the agitator shaft and the bearing and wear of the agitator shaft and the bearing due to the crystals can be prevented, leading to a reduction in the frequency of replacement of the agitator shaft and the bearing and a reduction in maintenance costs. In addition, when the compound is a polymerizable substance, polymerization and freezing due to sliding between the agitator shaft and the bearing can be sufficiently prevented. Thus, the above-described action of the agitator can be sufficiently kept, and a product can be stably obtained for a long period of time. Also, the pressure in the hydraulic wash column can be used to suitably transfer the mother liquor and/or melt to the bearing in the tank.

Suitable examples of a liquid flowed between the agitator shaft and the bearing include a mother liquor discharged from the hydraulic wash column and a circulation liquid containing a melt of the crystals discharged from the hydraulic wash column, which are described later.

The agitator may be any known agitator having a bearing. The agitator may be made of any material, and preferred examples of the material include metals such as stainless steel.

The length of the agitator shaft of the agitator is preferably 1/5 or more, more preferably 1/2 or more, still more preferably 4/5 or more, of the interior height of the tank.

The upper limit of the length of the agitator shaft is not limited, and it may be the same as the interior height of the tank.

The diameter of the agitator shaft can be appropriately selected according to the size of the agitator, and is preferably 10 to 500 mm, for example.

The diameter of the agitator shaft is preferably 1/1000 or more, more preferably 1/800 or more, of the inner diameter of the tank when the tank is viewed from the top.

The diameter of the agitator shaft is preferably 1/5 or less of the inner diameter of the tank.

The length of an agitator blade of the agitator (the distance from the agitator shaft to a tip of the blade) is preferably 1/10 or more, more preferably 1/8 or more, of the inner diameter of the tank when the tank is viewed from the top.

The length of the blade is typically 1/2 or less of the inner diameter of the tank.

Multiple agitator blades may be present in the axial direction. In other words, the agitator may have a multistage blade. When multiple agitator blades are present in the axial direction, any one of the agitator blades preferably has a length within the above-described preferred range of the inner diameter. More preferably, all of the agitator blades have a length within the above-described preferred range of the inner diameter.

The tank typically includes in the vicinity of the bottom a discharge port for discharging the slurry containing crystals of a compound from the tank.

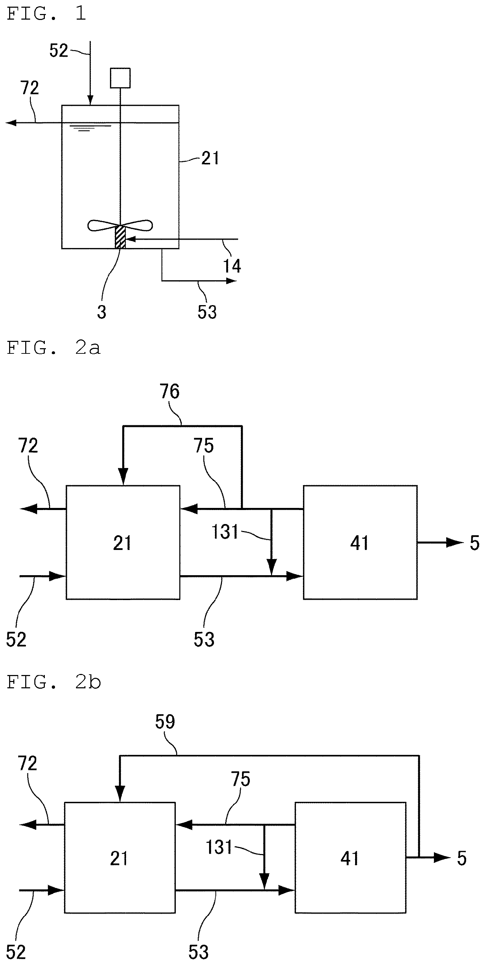

Although FIG. 1 and other figures, which are described later, show a tank including one discharge port that discharges the crystal-containing slurry, the tank may include multiple discharge ports.

The tank may have a baffle in the tank. The baffle is preferably provided in the direction from the top-roof side to the bottom side of the tank, for example.

Preferably, the baffle is provided on the bottom side of the tank.

The expression "the baffle is provided on the bottom side of the tank" refers to that the height of the center of gravity of the baffle is located at a position in the lower half of the inner volume of the tank.

Examples of a material for the baffle include metals such as stainless steel and resins.

The tank may include multiple baffles.

The retention time of the compound in the ripening tank may be appropriately adjusted according to the type of the compound to be purified. The retention time is preferably 0.5 to 6 hours in order to adjust the particle size distribution of the slurry to be sent to the wash column and to reduce the reflux ratio (flow rate of washing liquid/flow rate of purified compound) in the wash column. When the compound is a (meth)acrylic acid, the retention time is more preferably 1 to 5 hours, still more preferably 1.2 to 4.5 hours.

The retention time is a value calculated by dividing the volume of the suspension part in the ripening tank by the flow rate of the slurry fed from the ripening tank to the hydraulic wash column in the next step (subsequent stage).

The size of the crystallization tank is determined based on the required heat transfer area and the like. The retention time of the compound in the crystallization tank depends on the operating conditions.

Preferably, the tank further includes in the vicinity of the top roof a feed port for feeding the slurry containing crystals of a compound to the tank. This allows the slurry containing crystals of a compound to be suitably fed to the tank.

Although FIG. 1, which is described later, shows a tank 21 including one line 52 for sending a slurry to the tank and one slurry feed port, the tank 21 may include multiple lines 52 and multiple slurry feed ports. The nozzle of the line 52, constituting the feed port, may be bent at its tip to feed the slurry along the inner wall surface of the tank, or may be placed so that the tip is immersed in the liquid to feed the slurry into the liquid.

Preferably, the tank further includes in the vicinity of the top roof a discharge port for discharging the mother liquor in a supernatant part from the tank. The discharged mother liquor can be recycled. Thereby, the yield of the compound can be improved. For example, the discharged mother liquor can be returned to the tank in the previous step (preceding stage). The nozzle or pipe that constitutes the discharge port may be made of any material. Examples of the material include metals and alloys.

Although FIG. 1, which is described later, shows the tank 21 including one mother-liquor discharge port, the tank may include multiple discharge ports.

Herein, the supernatant part is a part where the slurry-derived mother liquor (supernatant) is present during use of the tank. The suspension part is a part where the slurry containing crystals of a compound (suspension) is present during use of the tank. The ranges of the supernatant part and the suspension part can be determined depending on the size, shape, location, and the like of the baffle.

The tank may further include a partition plate disposed between the feed port for the crystal-containing slurry and the discharge port for a mother liquor and disposed in the direction from the top-roof side to the bottom side of the tank. The partition plate further prevents crystals from entering the discharge port for a mother liquor.

The size of the partition plate can be appropriately selected according to the size of the tank.

Examples of a material for the partition plate include metals such as stainless steel and resins.

The tank may include one or two or more partition plates.

The tank may further include a weir in the vicinity of the top roof to prevent the crystals of a compound from entering the discharge port for a mother liquor.

The tank may include one weir. When the tank includes multiple mother-liquor discharge ports, each mother-liquor discharge port may be provided with a weir so that the tank includes multiple weirs, or a weir may be shared for multiple mother-liquor discharge ports.

The tank may have any size. For example, the tank preferably has an inner diameter of 100 to 50000 mm. The tank preferably has a height of 1000 to 100000 mm.

Instruments such as a thermometer, a pressure gauge, a liquid level gauge (e.g., of radar type), and a level switch (e.g., of float type) may be provide in the main body or periphery of the crystallization tank or the ripening tank. The ripening tank may include a sight glass (an observation window) in its side wall or the like. The sight glass may be covered with a cover. The ripening tank may include a hole such as a manhole or a hand hole (a hole for inserting a hand for maintenance) in its top roof, side wall, or the like. The ripening tank may include a rupture device or the like in its top roof or the like. The

### numbers of these are not limited.

### <Hydraulic wash column>

The purification apparatus of the present invention includes: a hydraulic wash column that includes a discharge port for a crystal-containing circulation slurry and a return port for a circulation liquid containing a melt of the discharged crystals; a pipe that feeds the crystal-containing slurry to the hydraulic wash column; a filter that filters the crystal-containing slurry in the hydraulic wash column; a pipe that is connected to the filter and discharges a mother liquor; and a unit that melts the crystals in the circulation slurry discharged from the discharge port.

The pipe that is connected to the filter and discharges a mother liquor is typically located above the filter. In a preferred embodiment of the purification apparatus of the present invention, at least a portion of the discharged mother liquor is flowed between the agitator shaft and the bearing in the tank. The rest of the discharged mother liquor can be reused, for example, as follows: the rest of the discharged mother liquor may be fed to the tank from the top-roof side, or may be mixed with the crystal-containing slurry to be fed into the hydraulic wash column and fed to the hydraulic wash column from the inlet thereof. In these ways, the slurry can be suitably fed into the hydraulic wash column. The compound is thus purified into a high-purity compound. The rest of the discharged mother liquor may be flowed into a nozzle provided on the hydraulic wash column to prevent freezing.

The filter may be made of any material and may be made of, for example, a metal such as stainless steel or a resin such as polytetrafluoroethylene (PTFE) or polyetheretherketone (PEEK), with the latter being preferred. The pipe may be made of any material and may be made of a metal or an alloy, for example.

The purification apparatus of the present invention may further include a dummy pipe that is connected to the filter that filters the crystal-containing slurry in the hydraulic wash column.

The dummy pipe is typically located below the filter. The dummy pipe may be made of any material and is preferably made of, for example, a resin such as polytetrafluoroethylene (PTFE), polyetheretherketone (PEEK), or a perfluoroalkoxy alkane (PFA).

Instruments such as a thermometer (e.g., of multipoint type), a pressure gauge, or an interface level meter (e.g., of optical type) may be disposed in the main body or periphery of the hydraulic wash column.

The purification apparatus of the present invention may further include: a discharge line that connects a discharge port (hereinafter also referred to as a product discharge port) for a crystal-containing slurry in the hydraulic wash column to a melting unit; and a return line that connects the melting unit to a return port for a circulation liquid containing the melt of the discharged crystals provided on the hydraulic wash column. The discharge line is preferably provided in the vicinity of the bottom of the hydraulic wash column. During use of the purification apparatus of the present invention, the circulation slurry or the circulation liquid containing a melt may be circulated in the discharge line and the return line. Herein, these circulation paths are also referred to as a melt loop.

In the circulation paths, the circulation slurry flows a portion between a point where the crystals in the hydraulic wash column are introduced into the circulation liquid and converted to a circulation slurry and a point where the crystals in the circulation slurry are melted. For example, in the melt loop, the circulation liquid returned from the return port in the bottom of the hydraulic wash column may be combined with the crystals in the hydraulic wash column and converted to a circulation slurry, and the circulation slurry may flow in a path (a discharge line) between the circulation-slurry discharge port and the melting unit.

The purification apparatus of the present invention may include a mechanism that discharges crystals from a crystal bed in the hydraulic wash column.

Non-limiting examples of the mechanism that discharges crystals from a crystal bed include a rotor blade or scraper described in JP 2005-509009 T and a mechanism using liquid dynamic pressure described in EP 1469926. One or more of these may be used. The rotor blade or scraper is preferably made of a metal such as stainless steel.

The melting unit is typically a heater. Examples of the heater include those having a structure that efficiently transfers heat to the crystal-containing slurry, such as a vertical multitubular heat exchanger, a horizontal multitubular heat exchanger, a double pipe heat exchanger, a spiral heat exchanger, a plate heat exchanger, a corrugated tube heat exchanger, and an electric heater. Preferably, the heater is provided in the melt loop, and the circulation slurry (the circulation liquid after melting) is circulated in the forced circulation system in which they are circulated by a pump provided in the melt loop.

The purification apparatus of the present invention may further include a mechanism (return mechanism) that returns a portion of the circulation liquid containing the melt obtained in the crystal melting unit to the hydraulic wash column.

The return mechanism may be any mechanism that is used to separate a portion of the circulation liquid from the rest and return the portion to the hydraulic wash column. For example, when the purification apparatus includes a product discharge line that is branched from the return line that connects the melting unit to the return port and is connected to the product discharge port, this branched line portion corresponds to the return mechanism. For example, the branched line portion may be a T-junction.

An example of the return mechanism is a mechanism that returns a portion of the circulation liquid containing the melt obtained in the crystal melting unit to the hydraulic wash column so that at least a portion of the portion of the returned circulation liquid serves as a washing liquid for crystals.

The return port is preferably provided at the bottom of the hydraulic wash column so that the circulation liquid can be returned upward.

The purification apparatus of the present invention may further include a mechanism for discharging a portion of the circulation liquid containing the melt obtained in the crystal melting unit and flowing the discharged liquid between the agitator shaft and the bearing in the tank. The mechanism is used to separate a portion of the circulation liquid from the rest and return the portion to the tank. For example, the mechanism includes a line for discharging the crystals of a compound from the hydraulic wash column, melting the crystals by heating to obtain a melt, and flowing a portion of the circulation liquid containing the melt between the agitator shaft and the bearing in the tank.

Herein, it can be said that the circulation liquid consists of the mother liquor and the melt.

The hydraulic wash column in the purification apparatus of the present invention may have any size. Preferably, the inner diameter of the column (the crystallization chamber) is 30 to 2000 mm, for example. The height of the column is preferably 1500 to 15000 mm.

The filter that filters the crystal-containing slurry in the hydraulic wash column may have any size.
Preferably, the inner diameter of the filter is 10 to 30 mm, for example. The height of the filter is preferably 20 to 300 mm.

The filter may be provided with a large number of circular holes, slits (notches), or rectangular holes, for example. The shape of the filter may be any shape and may be the same as the shape of the pipe, such as a cylindrical shape.

When the filter is provided with circular holes, the diameter of each hole may be appropriately adjusted depending on the size of the crystals, and is preferably 50 to 500 µm, for example. The number of holes is not limited, and may be adjusted according to the pressure loss, for example.

The pipe that is connected to the filter and discharges a mother liquor is typically located above the filter, as described above.

The number of pipes that are connected to the filter and discharge a mother liquor is not limited. For example, in an industrial-scale hydraulic wash column, 50 to 350 pipes are preferably connected in parallel per square meter of the cross-sectional area of the hydraulic wash column.

The purification apparatus of the present invention may further include a mechanism for heating the outer wall surface of the hydraulic wash column.

Non-limiting examples of the mechanism for heating the outer wall surface of the hydraulic wash column include a heating medium, a steam tracing system, an electric tracing system, and a known heater that adjusts the environmental temperature of the column. For example, part of the hydraulic wash column may be heated with a heating medium or the like. Preferably, substantially the entire hydraulic wash column is heated (jacket heating).

When the heating mechanism is a jacket-type heating mechanism, for example, the jacket may be made of any material such as a metal (e.g., stainless steel or carbon steel) or a resin.

The outside of the jacket may be provided with a heat insulating material, a tracing system, and the like. The structure of the jacket is not limited.

The inside of the jacket may be provided with any structure such as a structure that promotes heat transfer, such as a baffle.

The jacket preferably has an average thickness (the width of the space where the heating medium flows) of 5 to 200 mm, for example.

The heat flux through the wall of the hydraulic wash column from the jacket is preferably 100 W/m² or more, more preferably 200 W/m² or more, still more preferably 500 W/m² or more.

The upper limit of the heat flux through the wall of the hydraulic wash column from the jacket is typically 4000 W/m² or less, but is not limited thereto.

The difference between the melting point of the compound and the temperature of the heating medium fed to the jacket is preferably 1°C or more, more preferably 2°C or more, still more preferably 5°C or more. The upper limit is typically 20°C, but is not limited thereto.

A side wall of the jacket may be provided with a sight glass or a hand hole. In this case, they can be covered. Any numbers of sight glasses and hand holes may be provided.

Non-limiting examples of the heating medium include water, antifreeze, a methanol water mixture (an aqueous methanol solution), gas, and steam. The heating medium may be appropriately selected in consideration of the freezing point of the compound to be purified, for example.

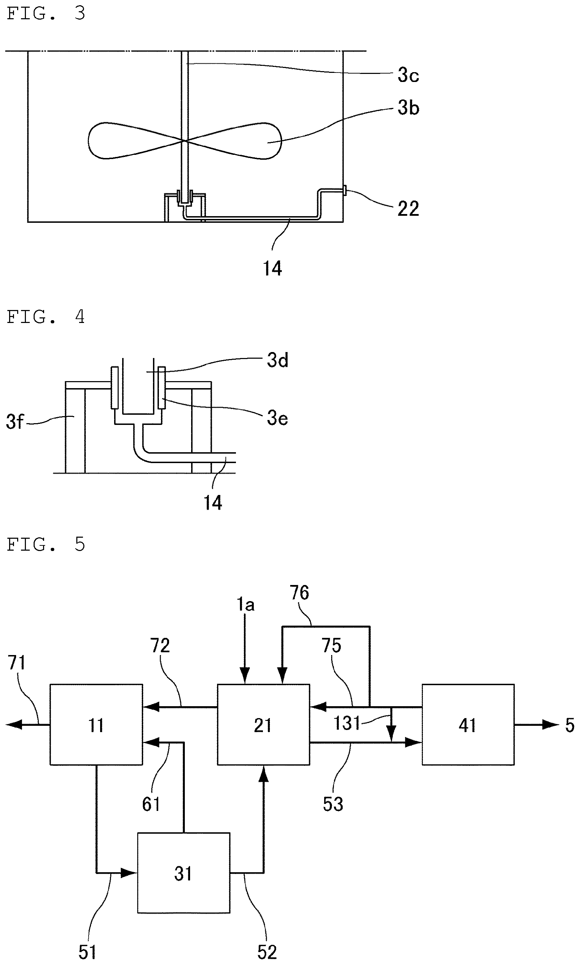

As described below, the number of pipes that feed the crystal-containing slurry to the hydraulic wash column and the number of feed nozzles (slurry feed ports) that may be connected to the tips of the pipes are not limited. Each of the numbers may be one or more (FIGS. 2a, 2b, and 5 show the case where the number of pipes that feed the crystal-containing slurry to the hydraulic wash column is one).

The feed nozzle may have, at its tip, a distribution mechanism that distributes the slurry.

The hydraulic wash column may further include a distribution chamber and a central displacer body (see JP 2005-509010 T).

### <Line for discharging slurry containing crystals of compound from tank and feeding the slurry to hydraulic wash column>

The purification apparatus of the present invention further includes a line for discharging the slurry containing crystals of a compound from the tank and feeding the slurry to the hydraulic wash column. For example, the line may include a pipe that extends from the discharge port for a slurry containing crystals of a compound of the tank to the feed port for the crystal-containing slurry of the hydraulic wash column; and an optional nozzle that is connected to the end of the pipe to constitute the discharge port for the slurry containing crystals of a compound of the tank and an optional nozzle that is connected to the end of the pipe to constitute the feed port for the crystal-containing slurry of the hydraulic wash column. The crystal-containing slurry may be discharged using a pump, for example. Preferred examples of the pump include a centrifugal pump, a diaphragm pump, and a rotary pump.

The number of the lines is not limited, and one or multiple lines may be present. (FIGS. 2a, 2b, and 5 show the case where the number of lines 53 that send a slurry from a (ripening) tank to the wash column is one.)

The nozzle constituting the feed port for the crystal-containing slurry of the hydraulic wash column may have, at its tip, a distribution mechanism that distributes the slurry.

### <Line for flowing between agitator shaft and bearing in tank mother liquor derived from slurry fed to hydraulic wash column, the slurry containing crystals of compound, and/or melt obtained by melting crystals>

The purification apparatus of the present invention further includes a line for flowing between the agitator shaft and the bearing in the tank a mother liquor derived from the slurry fed to the hydraulic wash column, the slurry containing crystals of a compound, and/or a melt obtained by melting the crystals.

Preferred examples of the line include: a line for discharging a mother liquor derived from the slurry containing crystals of a compound from the hydraulic wash column and flowing at least a portion of the discharged mother liquor between the agitator shaft and the bearing in the tank; and a line for discharging the crystals of a compound from the hydraulic wash column, melting the crystals by heating to obtain a melt, and flowing a portion of the circulation liquid containing the melt between the agitator shaft and the bearing in the tank.

The mother liquor derived from the slurry containing crystals of a compound is obtained by removing solids from the slurry fed to the hydraulic wash column, the slurry containing crystals of a compound. For example, the mother liquor can be obtained by removing solids from the slurry containing crystals of a compound by filtering, discharging the supernatant liquid, or other technique. Preferably, the mother liquor is obtained by removing solids at least by filtration. For example, as described above, the crystal-containing slurry may be subjected to filtration using a filter and the mother liquor may be discharged using a pipe connected to the filter.

The circulation liquid containing the melt circulates through the melt loop described above. This circulation liquid can be discharged from the melt loop and flowed between the agitator shaft and the bearing in the tank.

When the tank is a ripening tank, the purification apparatus of the present invention may further include a crystallization tank as the preceding stage of the ripening tank.

When the purification apparatus of the present invention further includes a crystallization tank, the purification apparatus of the present invention may include one or multiple crystallization tanks. When the purification apparatus of the present invention includes multiple crystallization tanks (first to N-th tanks), these crystallization tanks are preferably connected in series. In this case, the purification apparatus of the present invention typically includes a line for sending the slurry containing crystals of a compound from one crystallization tank to another crystallization tank optionally via a solid-liquid separator. In this case, in the purification apparatus of the present invention, at least one crystallization tank includes a line for feeding a liquid to be purified containing a compound. Preferably, in the purification apparatus of the present invention, at least the N-th crystallization tank includes a line for feeding the slurry containing crystals of a compound to the ripening tank.

When the purification apparatus of the present invention includes a crystallization tank and a ripening tank, at least one of the crystallization tank or the ripening tank is required to be the tank according to the present invention.

Preferably, the purification apparatus of the present invention further includes a line for sending out the product from the hydraulic wash column.

The purification apparatus of the present invention may further include a line for returning the mother liquor from a tank or unit in a subsequent stage to a tank or unit in a preceding stage.

The purification apparatus of the present invention may further include a mechanism that controls the amount of the slurry to be sent and the amount of the mother liquor to be returned. Examples of the control mechanism include valves attached to the lines.

The purification apparatus of the present invention may appropriately include different units commonly used in the purification apparatus.

The purification apparatus of the present invention may further include a mechanism for heating the entire or part of the outer wall surface of the tank or column in order to prevent freezing of the crystal-containing slurry and the like. The purification apparatus of the present invention may be placed in a temperature-controlled casing (including a large casing such as a building).

FIG. 1 is a schematic cross-sectional side view of an exemplary tank in the purification apparatus of the present invention. A crystal-containing slurry is fed into the tank 21 through the line 52 that sends the slurry to the tank. Next, the agitator shaft of the agitator in the tank 21 is rotated. Thereby, a suspension part is formed in at least a part of the tank, and in the suspension part, the crystal-containing slurry can be kept for a certain period of time, allowing the crystals in the slurry to grow. The crystal-containing slurry can then be discharged from a portion in the vicinity of the bottom of the tank through the line 53 that sends the slurry from the tank to the wash column.

The purification apparatus of the present invention shown in FIG. 1 includes a line 14 for flowing a mother liquor and/or a melt between the agitator shaft and the bearing in the tank. Through the line 14, the mother liquor and/or melt can be flowed into a bearing 3. Thereby, the crystal-containing slurry can be prevented from entering between the agitator shaft and the bearing (in the bearing); wear of the agitator shaft and the bearing due to the crystals can be prevented; and when the compound is a polymerizable substance, polymerization and freezing due to sliding between the agitator shaft and the bearing can be sufficiently prevented. Thus, a product can be stably obtained.

As shown in FIG. 1, the mother liquor can be recovered from the tank through a line 72 from a mother-liquor discharge port provided on the supernatant part. Here, a partition plate may be provided in the supernatant part to separate the nozzle, which is a slurry feed port, from the mother-liquor discharge port, thereby preventing crystals from entering the mother-liquor discharge port. The mother-liquor discharge port can be covered with a weir, thereby also preventing crystals from entering the mother-liquor discharge port.

FIG. 2a is a schematic diagram of exemplary lines to which the present invention is applicable, in the purification apparatus of the present invention. The crystal-containing slurry is discharged from the tank 21 and fed into a hydraulic wash column 41 through the line 53 for sending the slurry from the tank to the wash column.

In the hydraulic wash column 41, the crystals move downwards to form a crystal bed. The crystal bed is scraped off in the lower part of the column, suspended in a circulation liquid, and melted by heating. A portion of the circulation liquid containing the melt is sent out as a high-purity compound 5. A portion of the rest of the circulation liquid (washing liquid) is returned to the hydraulic wash column 41 and brought into countercurrent contact with the crystal bed to wash the crystals.

The inside of the hydraulic wash column 41 is provided with a filter that filters the crystal-containing slurry in the hydraulic wash column 41 and a pipe that is connected to the filter and discharges the mother liquor. Thereby, the mother liquor can be recovered from the crystal-containing slurry. In this way, a portion of the mother liquor derived from the slurry containing crystals of a compound is flowed between the agitator shaft and the bearing in the tank 21 through a line 76 for flowing the liquid between the agitator shaft and the bearing in the tank. In other words, the mother liquor is discharged from the mother-liquor discharge port through the line, and a portion of the mother liquor is fed between the agitator shaft and the bearing in the tank 21 through the line 76. The rest of the mother liquor is recovered through a line 75 for returning a portion of the discharged mother liquor to the tank from the top-roof side and a line 131 that sends a portion of the discharged mother liquor to the hydraulic wash column 41 again. Thereby, the rest of the mother liquor is reused. For example, the mother liquor returned through the line 75 can be fed to the tank 21 from the top roof of the tank 21 together with the slurry containing crystals of a compound. The mother liquor sent through the line 131 may be mixed with the slurry sent through the line 53, and the mixture may be fed into the hydraulic wash column 41 from the inlet thereof. Thereby, the mother liquor can be reused. The mother liquor sent through the line 131 can be used to suitably feed the slurry into the hydraulic wash column 41. The compound is thus purified into a high-purity compound. Furthermore, the rest of the mother liquor can be fed to a nozzle provided on the top roof of the hydraulic wash column 41 through a line that prevents freezing of the nozzle.

The mother liquor to be fed to the bearing may be heated before feeding. A heating mechanism may be present in the path of the line (line 76) that transfers the mother liquor to be fed to the bearing. The heating mechanism includes a mechanism including a heater through which the mother liquor is passed for heating, a mechanism directly heating the line that transfers the mother liquor so that the mother liquor passing through the line is heated, and/or a mechanism using both of these mechanisms. Examples of the heater include a vertical multitubular heat exchanger, a horizontal multitubular heat exchanger, a double pipe heat exchanger, a spiral heat exchanger, and a plate heat exchanger. An example of the mechanism for heating the mother liquor passing through the line by directly heating the line for transferring the mother liquor is a mechanism in which the line is provided with an electric heater, a steam trace system, a hot water trace system, a steam jacket, a hot water jacket, or the like. The whole or part of the line may be directly heated. Thereby, freezing in the bearing can be prevented.

In addition, since the mother liquor to be fed to the bearing is discharged from the hydraulic wash column in the subsequent stage, the mother liquor has a purity equal to or higher than the purity of the mother liquor in the tank and freezes easily (the freezing point is slightly higher). Therefore, preferably, the mother liquor is heated before feeding.

When a polymerizable material is purified, the mother liquor to be fed to the bearing typically contains an inhibitor since it is discharged from the hydraulic wash column in the purification apparatus. The mother liquor can sufficiently prevent polymerization.

Although not shown, the lines 75, 76, and 131, and the line for preventing freezing of the nozzle provided on the top roof of the hydraulic wash column may each be appropriately provided with a pump, a valve, an orifice, and/or a flow meter. Thereby, the amount of the mother liquor flowing through each line can be adjusted appropriately.

The crystals may be discharged from the discharge port at the bottom of the hydraulic wash column 41 and melted by heating, and a portion of the melt may be used as a product 5. The rest of the melt may be returned to the wash column as a washing liquid. If desired, a line may be provided that discharges crystals, melts the crystals by heating, and returns a portion of the melt to the wash column as a washing liquid.

FIG. 2b is a schematic diagram of other exemplary lines to which the present invention is applicable, in the purification apparatus of the present invention.

The purification apparatus shown in FIG. 2b includes a line 59 for flowing a portion of the liquid which is obtained by heating and melting crystals after discharging the crystals from the bottom of the hydraulic wash column between the agitator shaft and the bearing in the tank, instead of the line 76 for discharging the mother liquor derived from the slurry containing crystals of a compound from the top roof of the hydraulic wash column and flowing a portion of the discharged mother liquor between the agitator shaft and the bearing in the tank. Using such a purification apparatus, the liquid is flowed between the agitator shaft and the bearing in the tank. Thereby, the crystal-containing slurry can be prevented from entering between the agitator shaft and the bearing and wear of the agitator shaft and the bearing due to the crystals can be prevented. In addition, when the compound is a polymerizable substance, polymerization and freezing due to sliding between the agitator shaft and the bearing can be sufficiently prevented. Thus, the effects of the present invention can be achieved.

The liquid obtained through melting by heating may be further heated before feeding. A heating mechanism may be present in the path of the line (line 59) for flowing the liquid obtained by melting by heating between the agitator shaft and the bearing in the tank. The heating mechanism includes a mechanism including a heater through which the liquid obtained by melting by heating is passed for heating, a mechanism directly heating the line that transfers the liquid obtained by melting by heating so that the liquid passing through the line is heated, and/or a mechanism using both of these mechanisms. The heater and the heating mechanism are as described above.

In addition, since the liquid obtained by melting by heating, which is to be fed to the bearing, is discharged from the hydraulic wash column in the subsequent stage, the mother liquor has a purity equal to or higher than the purity of the mother liquor in the tank and freezes easily (the freezing point is slightly higher). Thus, the liquid is preferably heated before feeding.

FIG. 3 is a schematic side view of part of an agitator in the tank shown in FIG. 1. FIG. 3 shows that the line 14 passes through a feed port 22 and is connected to the bottom part of the bearing of the agitator. FIG. 3 shows a more specific example of the bearing 3 shown in FIG. 1.

FIG. 4 is a schematic cross-sectional side view of a bearing of the agitator shown in FIG. 1. A mother liquor is fed into a bearing 3e from the bottom-face side through the line 14, and can be flowed between a tip 3d of the agitator shaft and the bearing 3e. This can sufficiently prevent freezing of the liquid between the tip 3d of the agitator shaft and the bearing 3e. When the liquid between the tip 3d of the agitator shaft and the bearing 3e contains an easily-polymerizable component, the polymerization thereof can be sufficiently prevented. This can sufficiently prevent the agitator from being unusable due to clogging of the inside of the bearing 3e. In addition, the crystal-containing slurry can be prevented from entering between the tip 3d of the agitator shaft and the bearing 3e, and wear of the agitator shaft and the bearing due to the crystals can be prevented. As a result, a compound can be stably obtained over a long period of time.

FIG. 5 is another exemplary schematic diagram of lines to which the present invention is applicable, in the purification apparatus of the present invention. FIG. 5 illustrates a purification apparatus including one crystallization tank and one ripening tank as crystallizing units. The apparatus includes a line that directly sends a mother liquor from the ripening tank to the next downstream crystallization tank, and a line that directly discharges a residue (mother liquor) from the crystallization tank, which is the most downstream tank.

A compound solution 1a to be fed to the purification apparatus is introduced into a ripening tank 21. The solution 1a is cooled in a crystallization tank 11 that includes a cooling mechanism, and the slurry containing the precipitated crystals is sent to a solid-liquid separator 31 through a line 51. In the solid-liquid separator 31, the slurry is separated into a mother liquor and a concentrated crystal slurry. The concentrated crystal slurry is sent to the adjacent ripening tank 21 through the line 52, and the mother liquor is returned to the crystallization tank 11 through a line 61. A residue is discharged from the crystallization tank 11 through a line 71 to the outside of the purification apparatus. Thereby, the liquid level in the crystallization tank 11 is adjusted. The crystals are grown in the ripening tank 21, and then, the crystal-containing slurry is sent to the hydraulic wash column 41 through the line 53. In order to adjust the liquid level in the ripening tank 21, the mother liquor is directly sent from the ripening tank 21 to the crystallization tank 11 through the line 72.

The purification apparatus of the present invention is not limited the tank in use. The purification apparatus of the present invention may be any one having the above-described configuration and capable of flowing the mother liquor and/or melt between the agitator shaft and the bearing in the tank during use of the purification apparatus.

### (Method for producing compound of the present invention)

The present invention also encompasses a method for producing a compound, the method including: feeding a slurry containing crystals of a compound to a tank including an agitator having an agitator shaft and a bearing; discharging the slurry containing crystals of a compound from the tank and feeding the slurry to a hydraulic wash column; and flowing between the agitator shaft and the bearing in the tank at least one of a mother liquor derived from a slurry fed to the hydraulic wash column, the slurry containing crystals of a compound, or a melt obtained by melting the crystals; the tank being at least one of a crystallization tank that forms a slurry containing crystals of a compound or a ripening tank that is capable of keeping crystals of a compound suspended in the ripening tank.

In the method for producing a compound of the present invention, the feeding to a tank, feeding to the hydraulic wash column, and flowing are essentially performed in the stated order on a substance to be purified. (For example, as shown in FIG. 2a, a crystal-containing slurry is fed into the tank 21 through the line 52 that feeds the slurry to the tank. Next, the slurry containing crystals of a compound is discharged from the discharge port in the vicinity of the bottom of the tank, and is fed through the line 53 to the hydraulic wash column 41, for example. Then, the mother liquor derived from the slurry containing crystals of a compound is discharged from the mother-liquor discharge port in the vicinity of the top roof of the hydraulic wash column, and at least a portion of the discharged mother liquor is flowed through the line 76 between the agitator shaft and the bearing in the tank.) The following describes the feeding to a tank, feeding to the hydraulic wash column, and flowing in the stated order, followed by descriptions of agitating in the tank, discharging a mother liquor from the tank, and other steps. In the case of continuous purification, the steps are typically performed simultaneously when the purification apparatus is viewed as a whole.

Herein, the term "compound" refers to a compound obtainable by the production method of the present invention, and does not refer to raw materials, by-products, or solvents in the production method of the present invention. The term "compound" may also be referred to as a "target compound" or a "target substance". Herein, the term "impurities" refers to components other than the "compound", such as raw materials, by-products, and solvents.

### <Feeding to tank>

In the feeding to a tank, the slurry containing crystals of a compound is fed to a tank including an agitator having an agitator shaft and a bearing. The crystal-containing slurry is a suspension of the crystals of a compound and a mother liquor. In other words, the liquid portion of the slurry containing crystals of a compound to be fed to the tank is the mother liquor. The crystal-containing slurry can be obtained by forming crystals in a compound-containing solution (e.g., a (meth)acrylic acid aqueous solution or a crude (meth)acrylic acid solution) as described later. The compound-containing solution may be prepared in-house or procured from outside sources. The compound-containing solution and the mother liquor returned from the next step (e.g., the wash column) may be fed to the tank (e.g., a ripening tank). The compound-containing solution encompasses a crude compound.

In the feeding to a tank, the slurry containing crystals of a compound is preferably fed to the tank from a portion in the vicinity of the top roof of the tank. For example, the slurry containing crystals of a compound is preferably fed to the tank through a pipe or nozzle provided on the top roof of the tank.

In order to more stably obtain a product, the mass percentage of the crystals in the crystal-containing slurry to be fed to the tank is preferably 25% by mass or more, more preferably 30% by mass or more, still more preferably 35% by mass or more.

In order to achieve excellent fluidity of the slurry and to further reduce the risk of pipe clogging, the mass percentage of the crystals is preferably 55% by mass or less, more preferably 50% by mass or less, still more preferably 45% by mass or less.

The crystal-containing slurry to be fed to the tank may be one concentrated by a solid-liquid separator, for example.

Herein, in the case of a simple "crystal-containing slurry to be fed to the tank", the crystal-containing slurry to be fed to the tank refers to the crystal-containing slurry immediately before being fed to the tank, and, for example, refers to the crystal-containing slurry in a pipe or nozzle for feeding the crystal-containing slurry to the tank.

Preferably, in the crystal-containing slurry to be fed to the tank, the mother liquor contains the compound. Examples of the mother liquor include the compound and an aqueous solution of the compound. The mother liquor typically contains impurities other than the compound and water.

In the method for producing a compound of the present invention, the purity (mass percentage) of the compound in the mother liquor in the crystal-containing slurry to be fed to the tank is preferably 99% by mass or less.

Preferably, the mass percentage of the compound in the mother liquor is 80% by mass or more.

In the production method of the present invention, the compound is preferably an easily-polymerizable compound having a reactive double bond. Even in this case, the polymerization of the compound in the bearing can be sufficiently prevented.

In particular, in the production method of the present invention, the compound is more preferably an unsaturated carboxylic acid, still more preferably a (meth)acrylic acid, particularly preferably acrylic acid. Herein, the term "(meth)acrylic acid" refers to acrylic acid and/or methacrylic acid.

In the feeding to a tank, the crystal-containing slurry may be fed at any feed rate. For example, the feed rate is 0.2 × 10³ to 4.0 × 10⁵ kg/h in an industrial-scale tank.

In the feeding to a tank, the feed temperature of the crystal-containing slurry may be appropriately selected according to the melting point of the compound or the like. The feed temperature can be suitably adjusted to be approximately preferably -1°C to -15°C, more preferably - 1.5°C to -13.5°C, still more preferably -3.5°C to -12.5°C, particularly preferably -5°C to -11.5°C from the melting point of the pure substance of the compound.

For example, when the compound is a (meth)acrylic acid, the feed temperature of the crystal-containing slurry is preferably 0°C to 12°C, more preferably 1°C to 10°C, still more preferably 2°C to 8.5°C.

The feed temperature of the crystal-containing slurry is the temperature of the mother liquor in the crystal-containing slurry immediately before being fed to the tank (e.g., the crystal-containing slurry in the pipe or nozzle that feeds the crystal-containing slurry to the tank).

The tank may be operated with its interior being under increased pressure, atmospheric pressure, or reduced pressure. In order to suitably feed the mother liquor from the hydraulic wash column to the bearing in the tank, the tank is preferably operated at a pressure lower than the pressure in the hydraulic wash column, for example, at a pressure at least 0.01 MPa below the pressure in the hydraulic wash column.

### <Feeding to hydraulic wash column>

In the feeding to the hydraulic wash column, the slurry containing crystals of a compound is discharged from the tank and fed to the hydraulic wash column.

In the feeding to the hydraulic wash column, first, the slurry containing crystals of a compound is discharged from the tank. The slurry containing crystals of a compound is preferably discharged from a portion in the vicinity of the bottom of the tank.

The discharged slurry containing crystals of a compound is then fed to the hydraulic wash column. In the feeding to the hydraulic wash column, the slurry containing crystals of a compound is preferably fed to the hydraulic wash column from the top roof of the hydraulic wash column or from a portion in the vicinity of the top roof. For example, the slurry containing crystals of a compound is preferably fed to the hydraulic wash column through the pipe or nozzle provided on the top roof of the hydraulic wash column.

The feeding to the hydraulic wash column can be suitably performed using a pump such as a centrifugal pump, a diaphragm pump, or a rotary pump.

The mass percentage of the crystals in the crystal-containing slurry to be fed to the hydraulic wash column is preferably 1% by mass or more, more preferably 3% by mass or more, still more preferably 5% by mass or more.

The mass percentage of the crystals is preferably 50% by mass or less, more preferably 40% by mass or less, still more preferably 30% by mass or less, particularly preferably 20% by mass or less.

Herein, in the case of a simple "crystal-containing slurry to be fed to the hydraulic wash column", the crystal-containing slurry to be fed to the hydraulic wash column refers to the crystal-containing slurry immediately before being fed to the hydraulic wash column, and, for example, refers to the crystal-containing slurry in a pipe or nozzle that feeds the crystal-containing slurry to the hydraulic wash column.

Like the crystal-containing slurry to be fed to the tank, preferably, the crystal-containing slurry to be fed to the hydraulic wash column contains the compound in its mother liquor. Examples of the mother liquor include the compound and an aqueous solution of the compound. The mother liquor typically contains impurities other than the compound and water.

The preferred ranges of the purity of the compound, the mass percentage of water, the mass percentage of impurities other than the compound and water in the mother liquor are the same as the preferred ranges of the purity of the compound, the mass percentage of water, the mass percentage of impurities other than the compound and water in the mother liquor in the flowing step, which is described later.

In the feeding to the hydraulic wash column, the crystal-containing slurry may be fed at any feed rate. For example, the feed rate is 0.2 × 10³ to 4.0 × 10⁵ kg/h in an industrial-scale hydraulic wash column.

In the feeding to the hydraulic wash column, the feed temperature of the crystal-containing slurry can be appropriately selected according to the melting point of the compound or the like. For example, the feed temperature may be appropriately adjusted within the range of 0°C to 80°C.

For example, when the compound is a (meth)acrylic acid, the feed temperature of the crystal-containing slurry is preferably 5°C to 13°C, more preferably 6°C to 12°C.

The feed temperature of the crystal-containing slurry is the temperature of the mother liquor in the crystal-containing slurry immediately before being fed to the hydraulic wash column (e.g., the crystal-containing slurry in the pipe or nozzle that feeds the crystal-containing slurry to the hydraulic wash column).

### <Flowing>

In the flowing, a mother liquor derived from the slurry containing crystals of a compound to be fed to the hydraulic wash column and/or a melt obtained by melting the crystals is flowed between the agitator shaft and the bearing in the tank. For example, preferably, a mother liquor derived from the slurry containing crystals of a compound is discharged from the hydraulic wash column, and at least a portion of the discharged mother liquor is flowed between the agitator shaft and the bearing in the tank, or the crystals of a compound are discharged from the hydraulic wash column and melted by heating to obtain a melt, and a portion of the circulation liquid containing the melt is further discharged and is flowed between the agitator shaft and the bearing in the tank.

In the flowing, first, a mother liquor derived from the slurry containing crystals of a compound and/or a melt obtained by melting the crystals is discharged from the hydraulic wash column. Here, the mother liquor is preferably obtained by removing solids from the crystal-containing slurry in the hydraulic wash column by filtering, discharging the supernatant liquid, or other technique. In particular, the mother liquor is more preferably obtained by filtering the slurry using a filter and discharging the filtrate as a mother liquor using a pipe connected to the filter.

The filter may be made of any material and may be made of, for example, a metal such as stainless steel or a resin such as polytetrafluoroethylene (PTFE) or polyetheretherketone (PEEK), with the latter being preferred. The pipe may be made of any material and is preferably made of a metal or an alloy.

The mother liquor discharged in the mother-liquor discharging refers to the mother liquor in the pipe or nozzle immediately after being discharged in the mother-liquor discharging. For example, it is the mother liquor in the pipe immediately after passing through the filter.

During operation, the inside of the hydraulic wash column is essentially under pressure. The pressure is preferably within the range of 0.05 to 1.0 MPaG. The pressure can be used to suitably feed the discharged mother liquor and/or melt to the bearing in the tank. A pump or the like may be used as necessary.

In the flowing, at least a portion of the discharged mother liquor and/or melt is then flowed between the agitator shaft and the bearing in the tank. When a portion of the discharged mother liquor is flowed between the agitator shaft and the bearing in the tank, the rest of the discharged mother liquor is preferably reused. For example, the rest of the discharged mother liquor is recovered through the line 75 for returning the mother liquor discharged from the hydraulic wash column to the tank from the top-roof side or the line 131 that sends the mother liquor discharged from the hydraulic wash column to the hydraulic wash column again. Thereby, the rest of the mother liquor can be reused. For example, the mother liquor returned through the line 75 can be fed to the tank from the top roof of the tank together with the slurry containing crystals of a compound. The mother liquor passing through the line 131 is mixed with the slurry sent through the line 53, and the mixture is fed into the hydraulic wash column from the inlet thereof. Thereby, the mother liquor can be reused. Thus, the slurry can be suitably fed into the hydraulic wash column. The compound is thus purified into a high-purity compound. Furthermore, the rest of the mother liquor can be fed to the nozzle through the line for preventing freezing of the nozzle provided on the top roof of the wash column.

When a portion of the circulation liquid containing the discharged melt is flowed between the agitator shaft and the bearing in the tank, the rest of the circulation liquid can be discharged as a product or returned to the wash column as a washing liquid.

The mother liquor and/or melt, which does not substantially contain crystals, can sufficiently prevent wear of the agitator shaft and the bearing and is thus suitable as a flushing liquid. In addition, since the mother liquor and/or melt has a purity equal to or higher than that of the mother liquor in the tank, adverse effects on the quality can be sufficiently prevented. For example, when water is flowed between the agitator shaft and the bearing, impurities will increase. Furthermore, the mother liquor and/or melt is prone to freezing due to their high freezing point compared to the temperature in the tank. When the compound is a polymerizable substance, the mother liquor and/or melt typically contains an inhibitor and is difficult to polymerize.

During use of the purification apparatus of the present invention, the mother liquor and/or melt may be fed continuously or intermittently into the bearing.

The mass of the mother liquor flowed between the agitator shaft and the bearing in the tank is preferably 1/3 or less, more preferably 1/5 or less, still more preferably 1/7 or less, of the mother liquor discharged from the hydraulic wash column.

The mass of the mother liquor flowed between the agitator shaft and the bearing in the tank is preferably 1/10000 or more, more preferably 1/8000 or more, still more preferably 1/5000 or more, of the mother liquor discharged from the hydraulic wash column.

The flowing can be performed by flowing the mother liquor and/or melt from an opening provided on the bottom part or side part of the bearing and flowing the liquid between the agitator shaft and the bearing. In particular, in the flowing, the mother liquor and/or melt is preferably flowed from an opening provided at the bottom part of the bearing.

In the flowing, the mother liquor and/or melt may be fed to the bearing at any feed rate. The feed rate is 50 to 10000 kg/h in an industrial-scale tank, for example.

In the flowing, the mother liquor and/or melt is preferably flowed between the agitator shaft and the bearing at a linear velocity in the range of 0.01 to 30 m/s, more preferably in the range of 0.1 to 10 m/s.

The linear velocity can be determined as follows: the volume flow rate of the mother liquor and/or melt flowing through the line that feeds the mother liquor and/or melt to a bearing in the tank is measured with a flow meter and is divided by the cross-sectional area through which the mother liquor and/or melt flows between the agitator shaft and the bearing through the line.

In the flowing, the flow rate of the mother liquor and/or melt flowed between the agitator shaft and the bearing is preferably in the range of 0.001 to 20 m³/h, more preferably in the range of 0.1 to 3 m³/h.

The flow rate is the volume flow rate of the mother liquor and/or melt flowing through the line for feeding the mother liquor and/or melt to the bearing, and is measured with a flow meter.

In the flowing, the temperature at which the mother liquor and/or melt is fed to the bearing can be appropriately selected according to the melting point of the compound or the like. For example, the temperature can be appropriately adjusted within the range of 0°C to 80°C.

For example, when the compound is a (meth)acrylic acid, the temperature at which the mother liquor and/or melt is fed to the bearing is preferably 5°C to 13°C, more preferably 6°C to 12°C.

The mother liquor typically contains the compound, as described above. Examples of the mother liquor include a melt of the compound and an aqueous solution of the compound. The mother liquor typically contains impurities other than the compound and water.

In the method for producing a compound of the present invention, the purity (mass percentage) of the compound in the mother liquor is preferably 99% by mass or less, more preferably 98% by mass or less, still more preferably 97% by mass or less, particularly preferably 96% by mass or less.

The mass percentage of the compound in the mother liquor is preferably 85% by mass or more, more preferably 88% by mass or more, still more preferably 90% by mass or more.

The mass percentage of water in the mother liquor is preferably 0.1% by mass or more, more preferably 0.5% by mass or more, still more preferably 1% by mass or more.

The mass percentage of water in the mother liquor is preferably 8% by mass or less, more preferably 6% by mass or less, still more preferably 4% by mass or less.

The mass percentage of impurities other than the compound and water in the mother liquor is preferably 0.1% by mass or more, more preferably 0.4% by mass or more, still more preferably 0.8% by mass or more.

The mass percentage of impurities other than the compound and water in the mother liquor is preferably 8% by mass or less, more preferably 6% by mass or less, still more preferably 4% by mass or less.

When the compound is a (meth)acrylic acid, the impurities other than the compound and water may include acetic acid and furfural, for example.

In this case, the mass percentage of acetic acid in the mother liquor is preferably 0.1% by mass or more, more preferably 0.3% by mass or more, still more preferably 0.7% by mass or more.

The mass percentage of acetic acid in the mother liquor is preferably 8% by mass or less, more preferably 6% by mass or less, still more preferably 4% by mass or less.

When the compound is a (meth)acrylic acid, the mass percentage of furfural in the mother liquor is more preferably 0.01% by mass or more, more preferably 0.05% by mass or more, still more preferably 0.1% by mass or more.

The mass percentage of furfural in the mother liquor is preferably 2% by mass or less, more preferably 1% by mass or less, still more preferably 0.5% by mass or less.

The mother liquor is the mother liquor immediately before being fed to the bearing (e.g., the mother liquor in the line (pipe) that feeds the mother liquor to the bearing in the tank).

### <Agitating in tank>

The production method of the present invention may include agitating the slurry containing crystals of a compound in the tank.

In the agitating, the crystal-containing slurry is agitated typically using an agitator provided in the tank.

In the agitating, the rotation speed of the agitator is preferably in the range of 5 to 500 rpm, more preferably in the range of 10 to 300 rpm.

The agitating may be intermittent, preferably essentially continuous during use of the tank.

### <Discharging mother liquor from tank>

The production method of the present invention may include discharging a mother liquor in the supernatant part of the tank.

The discharged mother liquor can be recycled and reused. The discharged mother liquor may be fed to a unit in a preceding stage (e.g., the crystallization tank which is the preceding stage of the ripening tank) for reuse, for example. Thereby, the quality of the compound can be further improved.

The mother liquor may be discharged using a pump or the like.

### <Obtaining crystal-containing slurry>

The production method of the present invention preferably further includes obtaining a slurry containing crystals of a compound from a compound-containing solution.

The compound-containing solution is preferably a (meth)acrylic acid aqueous solution or a crude (meth)acrylic acid solution. The (meth)acrylic acid aqueous solution refers to a solution in which a (meth)acrylic acid is dissolved in water. The crude (meth)acrylic acid solution refers to a solution consisting of a (meth)acrylic acid and impurities such as by-products produced during the production of the (meth)acrylic acid. These can be obtained, for example, as follows: propylene and isobutylene are subjected to a vapor phase oxidation reaction to obtain a compound gas as a reaction product, and the compound gas is collected in an absorption column and optionally distilled. They are not limited to those synthesized in-house and may be procured from outside sources. The (meth)acrylic acid aqueous solution or the crude (meth)acrylic acid solution may be cooled, for example, to obtain a slurry containing (meth)acrylic acid crystals.

Examples of the impurities include acids such as propionic acid, acetic acid, maleic acid, benzoic acid, and acrylic acid dimers; aldehydes such as acrolein, furfural, formaldehyde, and glyoxal; acetone; and protoanemonin. In addition, solvents such as toluene and methyl isobutyl ketone may be contained.

Impurities in the compound-containing solution can be sufficiently removed by the production method of the present invention.

### <Obtaining compound-containing solution>

The production method of the present invention preferably further includes obtaining the compound-containing solution from a raw material.

The obtaining the compound-containing solution may be any process that can provide the compound-containing solution. When the compound is a (meth)acrylic acid, the obtaining the compound-containing solution can be suitably carried out by synthesizing acrylic acid, collecting the acrylic acid, and the like, as described in JP 2007-182437 A (Patent Literature 1), for example.

In the method for producing a compound of the present invention, the (meth)acrylic acid is preferably produced from at least one raw material selected from the group consisting of propane, propylene, acrolein, isobutene, methacrolein, acetic acid, lactic acid, isopropanol, 1,3-propanediol, glycerol, and 3-hydroxypropionic acid. The (meth)acrylic acid and/or the raw material may also be bio-based (meth)acrylic acids derived from renewable raw materials.

In the obtaining the compound-containing solution, impurities such as by-products are essentially formed. For example, when the compound is a (meth)acrylic acid, the impurities generated include water; acids such as propionic acid, acetic acid, maleic acid, benzoic acid, and acrylic acid dimers; aldehydes such as acrolein, furfural, formaldehyde, and glyoxal; acetone; methyl isobutyl ketone; toluene; and protoanemonin. Such impurities can be separated with excellent efficiency by a technique such as purification using the tank in the production method of the present invention. Thereby, a product can be efficiently obtained.

### (Method for purifying compound)

The present invention also encompasses a method for purifying a compound, the method including: feeding a slurry containing crystals of a compound to a tank including an agitator having an agitator shaft and a bearing; discharging the slurry containing crystals of a compound from the tank and feeding the slurry to a hydraulic wash column; and flowing between the agitator shaft and the bearing in the tank at least one of a mother liquor derived from a slurry fed to the hydraulic wash column, the slurry containing crystals of a compound, or a melt obtained by melting the crystals; the tank being at least one of a crystallization tank that forms a slurry containing crystals of a compound or a ripening tank that is capable of keeping crystals of a compound suspended in the ripening tank.

The purification method of the present invention can efficiently purify a compound.

Preferred embodiments of the purification method of the present invention are the same as the preferred embodiments of the production method of the present invention described above.

### EXAMPLES

The present invention will be described in more detail below with reference to examples, but the present invention is not limited by the following examples, and appropriate modifications may be made within the scope that can conform to the gist of the above and later descriptions. All of them are included in the technical scope of the present invention.
Unless otherwise specified, "%" indicates "% by mass" and "parts" indicates "parts by mass."

(Measurement instruments for gas chromatography and liquid chromatography)
Gas chromatograph: GC-2014 available from Shimadzu Corporation
Liquid chromatograph: LC-20AD HPLC unit available from Shimadzu Corporation

These instruments were used for measurement of acetic acid and furfural.

### (Method for preparing acrylic acid aqueous solution)

An acrylic acid aqueous solution was prepared according to the method described in WO 2010/032665 as follows: propylene was catalytically oxidized in vapor phase to obtain an acrylic acid-containing gas; and the acrylic acid-containing gas was treated in an absorption column.

### (Method for preparing feed slurry)

An acrylic acid aqueous solution was fed to the crystallization tank. A cooling medium was fed to a jacket provided around the wall of the crystallization tank for indirect cooling. The resulting crystals attached to the inner surface of the crystallization tank were scraped off with a scraper provided in the crystallization tank. Thus, a crystal-containing slurry (feed slurry) was prepared.

### (Purification apparatus)

A purification apparatus was used which is similar to or partially similar to the purification apparatus shown in FIGS. 2a, 3, and 4, including the following units:
the ripening tank 21 (inner diameter 5000 mm, inner height 10000 mm);
an agitator including an agitator shaft 3c (shaft diameter 135 mm, shaft length 10000 mm), an agitator blade 3b (agitator blade length 1425 mm, 2 stages), and the bearing 3 (inner diameter 135.4 mm, inner height about 150 mm) including, as shown in FIG. 4, a bottom part and a side part that cover the tip 3d of the agitator shaft, with the upper side being not covered, with the distance between the agitator shaft and the side part of the bearing 3 being 0.2 mm;
the hydraulic wash column 41;
the line 52 that feeds the slurry to the ripening tank 21;
the line 53 for feeding the slurry from the ripening tank 21 to the hydraulic wash column 41;
the line 72 for discharging a mother liquor in the supernatant part;
the line 75 for returning a portion of the discharged mother liquor to the ripening tank 21 from the top-roof side;
the line 76 for flowing a portion of the discharged mother liquor between the agitator shaft 3c and the bearing 3 in the ripening tanking 21; and
the line 131 that sends a portion of the discharged mother liquor to the hydraulic wash column 41 again.

### (Example 1)

A slurry containing acrylic acid crystals was fed from the top roof of the ripening tanking 21 and agitated using an agitator, and crystals were grown while the inside of the ripening tank 21 was kept in a suspension state.

Here, the mother liquor derived from the slurry containing crystals of a compound to be fed to the hydraulic wash column 41 was flowed between the tip 3d of the agitator shaft and the bearing 3e in the ripening tank 21 through the line 76 at a flow rate of 0.3 m³/h (the mass of the mother liquor is 1/400 of the mother liquor discharged from the hydraulic wash column), a linear velocity of 1.0 m/s, and a temperature of 11°C. The composition of the mother liquor flowed between the tip 3d of the agitator shaft and the bearing 3e was as follows: 94.7% by weight of acrylic acid, 1.8% by weight of acetic acid, and 0.1% by weight of furfural.

As a result, the agitator continued to operate normally without stopping.

### (Comparative Example 1)

A slurry containing acrylic acid crystals was fed to a ripening tank to grow crystals as in Example 1, except that no mother liquor was flowed between the agitator shaft and the bearing in the ripening tanking.

As a result, the agitator stopped due to the polymerization in the bearing, and the uniform suspension state in the ripening tank was lost, leading to the stoppage of the entire apparatus.

### REFERENCE SIGNS LIST

1a: solution of compound
3: bearing
3b: agitator blade
3c: agitator shaft
3d: tip of agitator shaft
3e: bearing
3f: base for bearing
5: product
11: crystallization tank
14: line for flowing mother liquor and/or melt between agitator shaft and bearing in (ripening) tank
21: (ripening) tank
22: feed port
31: solid-liquid separator
41: hydraulic wash column
51, 61, 71: lines
52: line that sends slurry to (ripening) tank
53: line for sending slurry from (ripening) tank to wash column
59: line for flowing portion of liquid between agitator shaft and bearing in (ripening) tank, liquid being obtained by discharging crystals from bottom of hydraulic wash column and melting crystals by heating
72: line for discharging mother liquor from supernatant part
75: line for returning portion of discharged mother liquor to (ripening) tank from top-roof side
76: line for flowing portion of discharged mother liquor between agitator shaft and bearing in (ripening) tank
131: line that sends portion of discharged mother liquor to hydraulic wash column again

## Claims

1. A purification apparatus for purifying a compound, the purification apparatus comprising:
a tank for use in the purification apparatus; and
a hydraulic wash column,
the tank being at least one of a crystallization tank that forms a slurry containing crystals of a compound or a ripening tank that is capable of keeping crystals of a compound suspended in the ripening tank,
the tank comprising an agitator having an agitator shaft and a bearing,
the purification apparatus further comprising:
a line for discharging the slurry containing crystals of a compound from the tank and feeding the slurry to the hydraulic wash column; and
a line for flowing between the agitator shaft and the bearing in the tank at least one of a mother liquor derived from the slurry fed to the hydraulic wash column, the slurry containing crystals of a compound, or a melt obtained by melting the crystals.

2. A method for producing a compound, the method comprising:
feeding a slurry containing crystals of a compound to a tank including an agitator having an agitator shaft and a bearing;
discharging the slurry containing crystals of a compound from the tank and feeding the slurry to a hydraulic wash column; and
flowing between the agitator shaft and the bearing in the tank at least one of a mother liquor derived from a slurry fed to the hydraulic wash column, the slurry containing crystals of a compound, or a melt obtained by melting the crystals;
the tank being at least one of a crystallization tank that forms a slurry containing crystals of a compound or a ripening tank that is capable of keeping crystals of a compound suspended in the ripening tank.

3. The method for producing a compound according to claim 2,
wherein the compound is a (meth)acrylic acid.

4. The method for producing a compound according to claim 3,
wherein the (meth)acrylic acid is produced from at least one raw material selected from the group consisting of propane, propylene, acrolein, isobutene, methacrolein, acetic acid, lactic acid, isopropanol, 1,3-propanediol, glycerol, and 3-hydroxypropionic acid.

5. A method for purifying a compound,
the method comprising:
feeding a slurry containing crystals of a compound to a tank including an agitator having an agitator shaft and a bearing;
discharging the slurry containing crystals of a compound from the tank and feeding the slurry to a hydraulic wash column; and
flowing between the agitator shaft and the bearing in the tank at least one of a mother liquor derived from a slurry fed to the hydraulic wash column, the slurry containing crystals of a compound, or a melt obtained by melting the crystals;
the tank being at least one of a crystallization tank that forms a slurry containing crystals of a compound or a ripening tank that is capable of keeping crystals of a compound suspended in the ripening tank.
